# EUROPÄISCHE PATENTSCHRIFT

(11) **EP 0 998 351 B1**
(45) Veröffentlichungstag und Bekanntmachung des Hinweises auf die Patenterteilung: **30.05.2001**
(21) Anmeldenummer: 98954341.8
(22) Anmeldetag: 05.10.1998
(51) Int. Cl.: B01J 31/02

(54) **VERWENDUNG VON TRIS(TRIFLUOROMETHYLSULFONYL)METHAN UND DESSEN ALKALI- UND ERDALKALIMETALLSALZEN ALS KATALYSATOREN BEI C-C VERKNÜPFENDEN SYNTHESEN UND DAS NEUE Mg-SALZ VON TRIS(TRIFLUOROMETHYLSULFONYL)METHAN**
USE OF TRIS (TRIFLUORO METHYLSULFONYL) METHANE, IN ADDITION TO ALKALINE METAL SALTS AND ALKALINE-EARTH METAL SALTS THEREOF AS CATALYSTS DURING C-C BONDING SYNTHESES AND THE NOVEL Mg SALT OF TRIS (TRIFLUORO METHYLSULFONYL) METHANE
UTILISATION DE TRIS (TRIFLUORO METHYLSULFONYL) METHANE ET DE SES SELS ALCALINS ET ALCALINOTERREUX COMME CATALYSEURS DANS DES SYNTHESES DE LIAISON C-C ET LE NOUVEAU SEL MG DE TRIS (TRIFLUORO METHYLSULFONYL) METHANE

(30) Priorität: 06.10.1997 DE 19743985
(43) Veröffentlichungstag der Anmeldung: 10.05.2000
(73) Patentinhaber: MERCK PATENT GmbH, 64293 Darmstadt (DE)
(72) Erfinder: BUCHHOLZ, Herwig, D-60599 Frankfurt (DE); FRANZ, Klaus-Dieter, D-65779 Kelkheim (DE); MAYR, Herbert, D-82319 Starnberg (DE); FUNKE, Marcus-Alexander, D-64289 Darmstadt (DE); ZEHETNER, Andrea, D-80996 München (DE)
(86) Internationale Anmeldenummer: EP9806326
(87) Internationale Veröffentlichungsnummer: WO9917878

(56) Entgegenhaltungen:
- US-A- 5 554 664
- CHEMICAL ABSTRACTS, vol. 109, no. 1, 4. Juli 1988 Columbus, Ohio, US; abstract no. 6063p, TUROWSKY L.: "Tris[(trifluoromethyl)sulfonyl]methane, HC(SO2CF3)3" Seite 572; Spalte 2; XP002091455 in der Anmeldung erwähnt & INORG. CHEM., Bd. 27, Nr. 12, Seiten 2135-2137,

## Beschreibung

Die Erfindung betrifft die Verwendung von Tris(trifluoromethylsulfonyl) methan und dessen Alkali- und Erdalkalimetallsalzen als Katalysatoren bei der Umsetzung von durch Lewis-Säuren bzw. Brönsted-Säuren aktivierbaren Elektophilen mit Nucleophilen oder zur Durchführung von [4+2]-Cycloadditionen sowie Bereitstellung des neuen Mg- Salzes von Tris(trifluormethylsulfonyl)methan.

Die Lewis-Säure induzierte Umsetzung von Elektrophilen, wie z. B. Alkylchloriden oder Carbonylverbindungen, mit Nucleophilen stellt eine einfache, weit verbreitete und seit langem bekannte Synthesemethode zum Aufbau von Kohlenstoffgerüsten dar.

Die dabei als Lewis-Säuren eingesetzten Metallhalogenide MXₙ, wie z. B. TiCl₄, ZnCl₂ oder AlCl₃, werden oft, wie im Falle von Friedel-Crafts-Acylierungen, in äquimolaren Mengen eingesetzt und müssen bei der Aufarbeitung der Reaktionsansätze durch Hydrolyse irreversibel desaktiviert werden. Dabei fallen große Mengen nichtverwertbarer Abfälle an. Die Durchführung von Synthesen im großtechnischen Maßstab, bei denen nicht verwertbare Nebenprodukte entstehen, ist aber aus ökologischer und ökonomischer Sicht sehr problematisch. Wiederverwertbaren, Lewis-aciden Systemen und deren Einsatz bei Lewis-Säure induzierten CC-Verknüpfungen ist, wenn immer möglich, der Vorzug zu geben.

Wiedereinsetzbare Lewis-Säure-Systeme in C-C verknüpfenden Synthesen stellen z. B. Lithiumperchlorat oder Magnesiumperchlorat in stark koordinierenden organischen Lösungsmitteln, wie Diethylether, oder in nicht koordinierenden Lösungsmitteln, wie Dichlormethan, dar.

Dabei zeigen die Lösungen von Lithiumperchlorat oder Magnesiumperchlorat in Diethylether ein ausreichendes lonisationsvermögen, um z. B. Tritylchlorid partiell zu ionisieren, um die gewünschte Umsetzung zu erzielen, wobei das lonisationsvermögen mit zunehmender Lithiumsalzkonzentration zunimmt. (vgl. Y. Pocker, R. F. Buchholz, J. Am. Chem. Soc. 1970, 92, 2075-2084). Die starken koordinativen Wechselwirkungen zwischen dem Lithiumkation und dem Lösungsmittel Diethylether begünstigen aber die Ausbildung von Etheraten und vermindern daher die Lewis-Acidität des Lithiumkations erheblich (Y. Pocker et al., a.a.O). Daher bleiben die ausgeprägten Lewis-aciden Eigenschaften von Lithiumperchloratlösungen in Ether auf hochkonzentrierte Lösungen beschränkt. Derartige Lösungen finden insbesondere bei der Katalyse von Diels-Alder-Reaktionen mit hydrolyseempfindlichen Substanzen Anwendung, wobei als zusätzlicher Effekt die Bildung der endo-Cycloaddukte begünstigt wird.

Obwohl die Löslichkeit von Lithiumperchlorat in nicht koordinierenden Lösungsmitteln wie Dichlormethan sehr gering ist (Löslichkeit <<10⁻³ mol/l), besitzt auch eine Suspension von Lithiumperchlorat in Dichlormethan Lewis-acide Eigenschaften. Aldehyde werden beispielsweise für Reaktionen mit 1-tert.-Butyldimethylsiloxy-1-methoxy-ethen zu den entsprechenden β-Siloxycarbonsäureestern (Mukaiyama-Aldolreaktion) in diesem heterogenen Medium ausreichend aktiviert, wobei Lithiumperchlorat in katalytischen Mengen bezüglich der Edukte eingesetzt wird.

Der Einsatz von Lithiumperchlorat und Magnesiumperchlorat als Lewis-Säuren in der organischen Synthese stellt jedoch ein erhebliches Gefahrenpotential dar, das einerseits durch die Redoxinstabilität der Perchlorate (Zersetzungs-und Explosionsgefahr) und andererseits durch die nicht sicher auszuschließende Bildung organischer Perchlorate bei der zu katalysierenden Reaktion begründet ist (P. G. Urben, Chemtech 1991 (5), 259; A. B. Charette in Encyclopedia of Reagents for Organic Synthesis (Hrsg. L. A. Paquette), J. Wiley and Sons, New York 1995 Vol. 5, S. 3155). Der Einsatz von Perchloraten als Lewis-Säuren in der industriellen Synthese ist daher problematisch. Zwar ist die Rückgewinnung von Lithiumperchlorat oder Magnesiumperchlorat aus den organischen Reaktionslösungsmitteln, wie Ether, Dichlormethan oder Pentan, im Prinzip durch wäßrige Extraktion möglich, jedoch ist die Kristallisation der Perchlorate aus Wasser wiederum mit den vorstehend beschriebenen Gefahren und zudem mit einem hohen Energieaufwand verbunden, so daß die Aufarbeitung aus sicherheitstechnischen und wirtschaftlichen Gründen ungünstig ist.

Der vorliegenden Erfindung liegt daher die Aufgabe zugrunde, Lewis-Säure-Katalysatoren für die Umsetzung von Elektrophilen mit Nucleophilen bereitzustellen, die die gefahrlos industriell eingesetzt und leicht und gefahrlos ohne hohen Energieaufwand zurückgewonnen werden können.

Diese Aufgabe wird gelöst durch die Verwendung von wenigstens einer Verbindung der Formel (1)

M^{+x}[C(SO₂CF₃)₃]ₓ (1)

wobei x 1 oder 2 ist,
M ein Wasserstoff- oder Alkalimetallatom bedeutet, wenn x für 1 steht, bzw. ein Erdalkalimetallatom bedeutet, wenn x für 2 steht,
als Katalysator bei C - C werkwüpfenden Synthesen.

Die Herstellung von Tris(trifluormethylsulfonyl)methan sowie von bestimmten Alkali- und Erdalkali-tris(trifluormethylsulfonyl)methaniden der Formel (1) ist bekannt und in L. Turowski, K. Seppelt, Inorg. Chem. 1988, 27, 2135-2137; K. Seppelt, Angew. Chem. 1993, 105, 1074-1076 und in J. Org Chem. Vol 38, No. 19, Seiten 3358 ff. beschrieben.

Tris(trifluomethylsulfonyl)methan sowie dessen Lithium- und Kaliumsalz sind auch aus (Beilstein-Registrier-Nummern 4767561, 5899593, WO 92/FR1024) bekannt.

Die Verbindungen der Formel (1) lassen sich wegen ihrer geringen Löslichkeit in nicht-koordinierenden Lösungsmitteln einfach durch Filtration aus den Reaktionsansätzen entfernen. Da sich die Verbindungen der Formel (1) durch hohe Redoxstabilität und thermische Stabilität auszeichnen, besitzen Synthesen mit diesen Salzen und ihre Wiedergewinnung aus den Reaktionsansätzen ein deutlich niedrigeres Gefahrenpotential als analoge Reaktionen mit anorganischen Perchloraten.

Als Verbindung der Formel (1) werden bevorzugt die Säure selbst (M=H), die Lithium-, Kalium-, Magnesium- oder Calciumsalze, und insbesondere die Lithium- und Magnesiumsalze verwendet.

Die Verbindungen der Formel (1) werden in homogener Lösung in koordinierendem, organischem Lösungsmittel, wie z. B. Diethylether, THF oder Dioxan verwendet. Die Konzentration der Verbindung (1) liegt dabei in katalytischen Mengen vor, bevorzugt im Bereich von 0,01 bis 0,1 mol/l, insbesodere 0,02-0,04 mol/l.

Die Verwendung der Verbindungen der Formel (1) als Suspension in nicht koordinierenden, organischen Lösungsmitteln, wie z. B. Dichlormethan, Chloroform, Tetrachlorkohlenstoff oder Kohlenwasserstoffen, wie n-Hexan, n-Pentan, bevorzugt Dichlormethan, ist ebenso möglich.

Bezogen auf Elektrophil können 0,01 - 10 mol%, vorzugsweise 0,5- 6 mol% einer Verbindung der Formel (1) eingesetzt werden.

Mit Hilfe der Verbindungen der Formel (1) können durch Lewis-Säuren bzw. Brönsted-Säuren induzierte C-C verknüpfende Synthesen und [4+2] Cycloadditionen jeder Art katalysiert werden.

Um die dabei erhaltenen Produkte mit einer ausreichend hohen Ausbeute und vertretbaren Reaktionsgeschwindigkeiten zu erhalten, ist es vorteilhaft, bestimmte Mindestwerte sowohl bei der relativen Reaktivität der zur Umsetzung kommenden elektrophilen Verbindungen gegenüber den nucleophilen Verbindungen als auch der Nucleophilie N der zum Einsatz nucleophilen Verbindungen nicht zu unterschreiten.

Der Parameter N für die Nucleophilie einer Verbindung wurde von Mayr und Patz, Angew. Chem. 1994, 106, 990-1010 eingeführt.

Für zahlreiche nucleophile Verbindungen kann der Wert N dieser Veröffentlichung, insbesondere Seiten 1003-1004 entnommen werden. Tabelle 1 enthält auch eine Reihe von N-Werten.

Die relative Reaktivität von elektrophilen Verbindungen kann u. a. mit Hilfe der Ethanolysegeschwindigkeitskonstante K_{EtOH}, d. h. der Solvolyse- Geschwindigkeitskonstante in 100% Ethanol bei 25°C angegeben werden.

Insbesondere für die Angabe der relativen Reaktivität von Alkylhalogeniden, insbesondere Chloriden oder -Bromiden, kann diese Bestimmungsgröße herangezogen werden. Die entsprechenden Werte für k_{EtOH} (25°C) für zahlreiche Alkylhalogenide können z. B. der Veröffentlichung von J. P. Dau-Schmidt, und H. Mayr in Chem. Ber. 1994, 127, Seiten 205-212 oder der Dissertation von J. P. Dau-Schmidt, Medizinische Universität Lübeck 1992 entnommen werden.

Dabei eignen sich für eine mit dem Lithiumsalz gemäß Formel (1) in einer Suspension in nicht koordinierenden, organischen Lösungsmitteln, vorzugsweise in Dichlormethan, induzierte Umsetzung von Alkylhalogeniden, insbesondere von Alkylchloriden oder Alkylbromiden, solche mit einem k_{EtOH} (25°C)-Wert von ≥ 5 x 10⁻⁵ s⁻¹ bzw. mit dem Lithiumsalz gemäß Formel (1) in einer ≥ 0,03 molaren Lösung in koordinierenden, organischen Lösungsmitteln, vorzugsweise in Diethylether, Alkylhalogenide, insbesonderer Alkylchloride oder Alkylbromide, mit einem k_{EtOH} ≥ 1 x 10⁻³ S⁻¹.

Bei Verwendung des Magnesiumsalzes gemäß Formel (1), in nicht koordinierenden, organischen Lösungsmitteln, vorzugsweise in Dichlormethan, suspensiert, ist es vorteilhaft Alkylhalogenide, insbesondere Chloride oder Bromide, mit einem k_{EtOH} (25°C)-Wert von ≥ 1 x 10⁻⁷ s⁻¹ einzusetzen.

Die entsprechenden zur Reaktion mit den Alkylhalogeniden kommenden nucieophilen Verbindungen sollten in jedem Fall einen Wert N von ≥ 1,62 aufweisen.

Beispiele für Alkylhalogenids, vorzugsweise Alkylchloride oder Alkylbromide, mit den angegebenen Mindestwerten an k_{EtOH}, die jedoch keine erschöpfende Aufzählung darstellen, sind Alkylhalogenide, wobei Alkyl vorzugsweise für geradkettiges oder verzweigtes C₁-C₁₀-Alkyl steht, das gegebenenfalls durch 1 bis 3 gleiche oder verschiedene beliebige Reste am α-Kohlenstoffatom substituiert sein kann, insbesondere durch Aryl, bevorzugt C₆-C₁₀-Aryl, substituiertes Aryl, z. B. Methoxyphenyl, Tolyl, Cumenyl, Aminophenyl, Di- oder Trialkylaminophenyl, C₂-C₆-Alkenyl, z. B. 1-Methyl-2-butenyl, C₅-C₆-Cycloalkenyl, insbesondere Cyclopentenyl und 1,1-Dimethyl-2-butinyl, 3-Pentan-2-yl. Halogenid steht für Fluorid, Chlorid, Bromid oder lodid, bevorzugt für Chlorid und Bromid, besonders bevorzugt für Chlorid.
Nachfolgend wird eine Aufstellung (1.1.) von ausgewählten Alkylchloriden, die in einer Suspension von LiC(SO₂CF₃)₃ in Dichlormethan als mögliche Reaktionspartner von Allyltrimethylsilan oder reaktiveren Nucleophilen (N ≥1,62) in Frage kommen, gegeben:

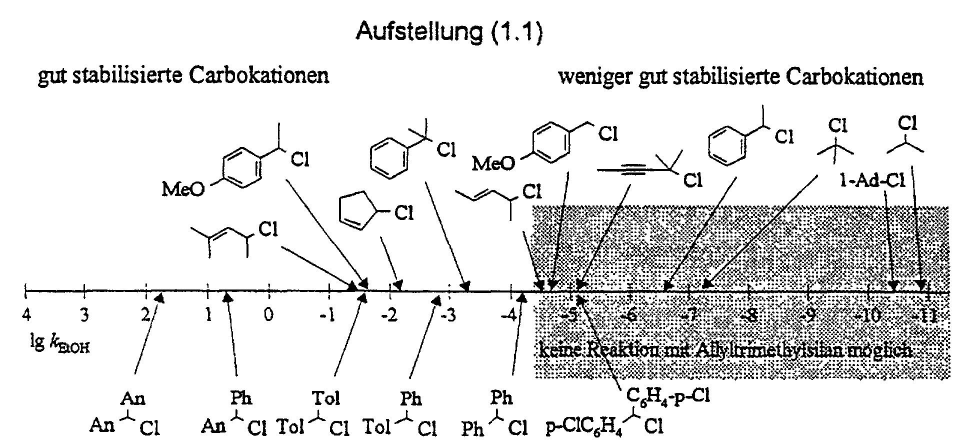

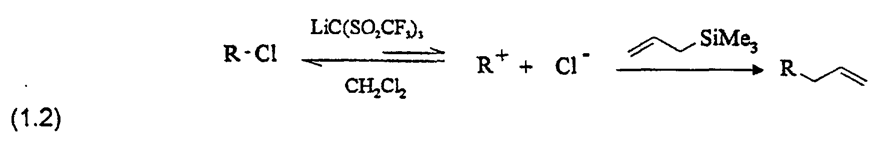

Entsprechend dem vorstehenden Reaktionsschema (1.2) ergeben Alylchloride mit einer Ethanolysegeschwindigkeitskonstante k_{EtOH} ≥ 5 x 10⁻⁵ s⁻¹ gut stabilisierte Carbokationen mit den erfindungsgemäß verwendeten Li-Salzen der Formel (1) und sind daher zur Umsetzung mit nucleophilen Verbindungen, deren Wert N ≥ 1,62 ist, geeignet.

Ausgewählte Alkylchloride, die in einer 0,03 M Lösung von LiC(SO₂CF₃)₃ in Diethylether als mögliche Reaktionspartner von Allyltrimethysilan oder reaktiveren Nucleophilen (N≥ 1,62) in Frage kommen, sind nachstehender Aufstellung (1.3) zu entnehmen:

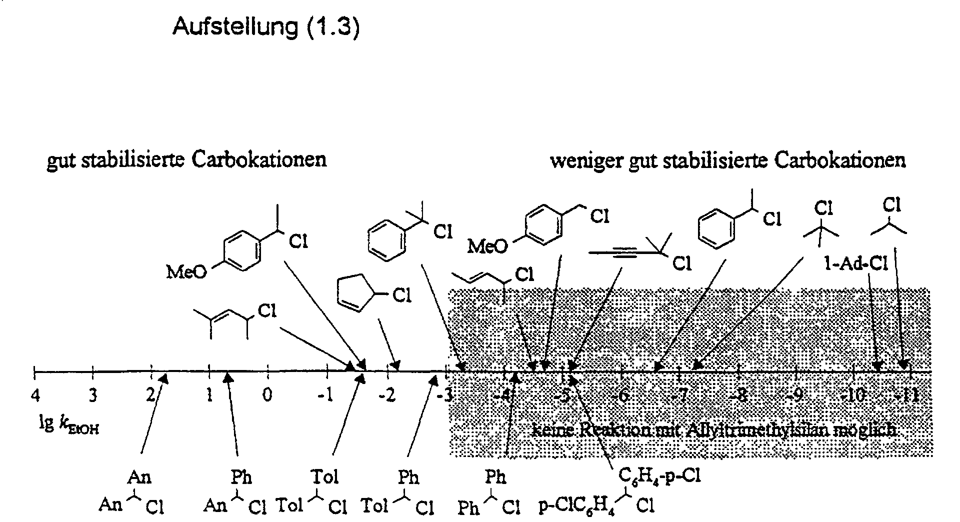

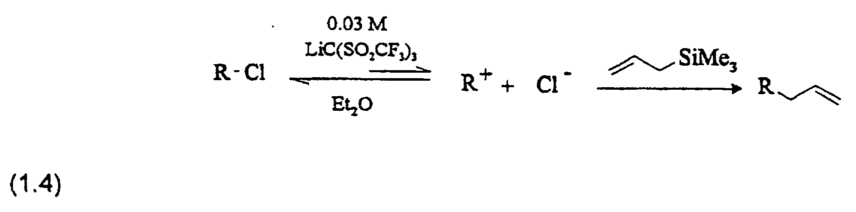

Entsprechend dem Reaktionsschema (1.4) ergeben Alkylchloride mit einer Ethanolysegeschwindigkeitskonstante k_{EtOH} (25°C) ≥ 1 x 10⁻³ s⁻¹ mit den erfindungsgemäß verwendeten Li-Salzen der Formel (1) gut stabilisierte Carbokationen, die mit nucleophilen Verbindungen, deren N ≥ 1,62 ist, umgesetzt werden können.

Ausgewählte Alkylchloride, die in einer Suspension von Mg(C(SO₂CF₃)₃)₂ in Dichlormethan als mögliche Reaktionspartner von Allyltrimethylsilan oder reaktiveren Nucleophilen (N ≥ 1,62) in Frage kommen, sind der nachfolgenden Aufstellung (1.5) zu entnehmen:

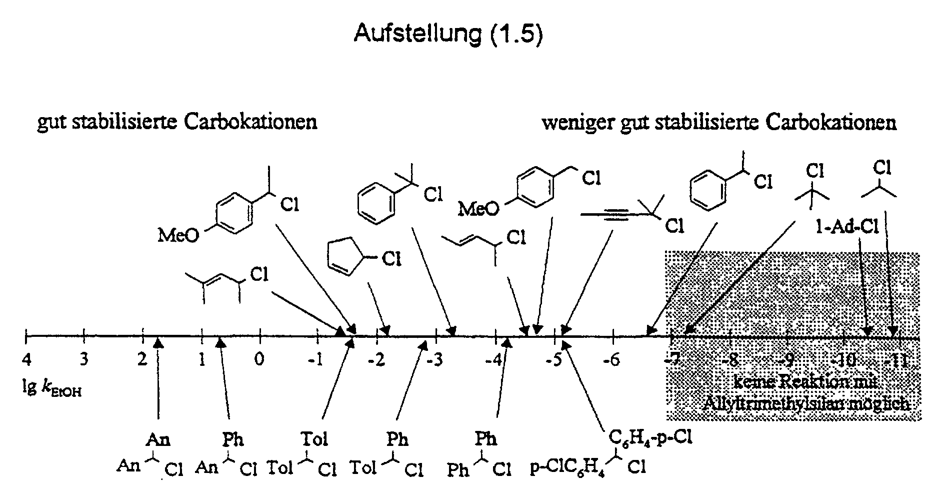

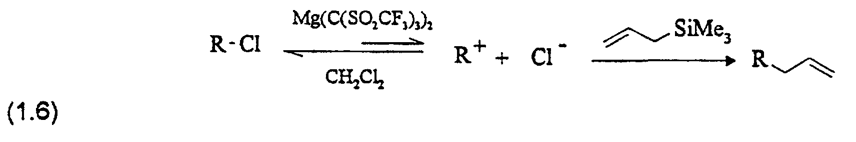

Entsprechend dem vorstehenden Reaktionsschema (1.6) ergeben Alkylchloride mit einer Ethanolysegeschwindigkeitskonstante k_{EtOH} (25°C) ≥ 1 x 10⁻⁷ s⁻¹ mit den erfindungsgemäß zum Einsatz kommenden Mg-salzen der Formel (1) gut stabilisierte Carbonkationen, die mit nucleophilen Verbindungen, deren N ≥ 1,62 ist, gut reagieren.

Die relative Reaktivität einer elektrophilen Verbindung gegenüber einer nucleophilen Verbindung bei der erfindungsgemäß induzierten Reaktion kann auch durch Angabe der relativen Reaktivitätskonstante kᵣₑᵢ erfolgen. Durch kᵣₑᵢ werden z. B. die relativen Reaktivitäten von Acetalen und Alkylethern gegenüber Allyltrimethylsilan in Dichlormethan in Anwesenheit katalytischer Mengen ZnCl₂-Etherat beschrieben. Entsprechende Werte können H. Mayr, J.-P. Dau-Schmidt, Chem. Ber. 1994, 127, 213-217 entnommen werden.

Für die erfindungsgemäß mit dem Lithiumsalz Formel (1), suspendiert in nicht koordinierenden Lösungsmitteln, vorzugsweise in Dichlormethan, als Katalysator induzierten Umsetzungen eignen sich vorzugsweise Acetale oder Alkylether, vorzugsweise Methylether, deren relative Reaktivitätskonstante kᵣₑᵢ gleich oder größer der von Benzaldehyddimethylacetal ist. Beim Einsatz des Magnesiumsalzes gemäß Formel (1) sollten die Acetale bzw. Alkylether, vorzugsweise Methylether, eine relative Reaktivitätskonstante kᵣₑᵢ aufweisen, die gleich oder größer der von 1-Tolyl-1-methoxy-ethan ist, wobei die nucleophile Verbindung in jedem Fall einen Wert N ≥ 1,62 aufweisen sollte.

Dialkylacetale beliebiger Ketone oder Aldehyde, insbesondere C₁-C₆-Alkylacetale, besonders bevorzugt Dimethylacetale, gemischte und cyclische Dialkylacetale können eingesetzt werden, sofern sie die angegebene relative Reaktivitätskonstante kᵣₑᵢ aufweisen. Dies gilt auch für Alkylether beliebiger Hydroxyverbindungen, wobei Alkyl, vorzugsweise für C₁-C₆-Alkyl, insbesondere Methyl oder Ethyl, steht, oder für N,O-Acetale, d. h. für Reaktionsprodukte beliebiger disubstituierter Amine, wie beispielsweise Diisopropylamin oder Ethylphenylamin, mit Alkoholen und Aldehyden, insbesondere Formaldehyd.

Nachfolgende Aufstellung (1.7) gibt ausgewählte Methylether und Acetale an, die als mögliche Reaktionspartner von Allyltrimethylsilan oder reaktiveren Nucleophilen (N ≥ 1,62) in einer Suspension aus LiC(SO₂CF₃)₃ in Dichlormethan in Frage kommen.

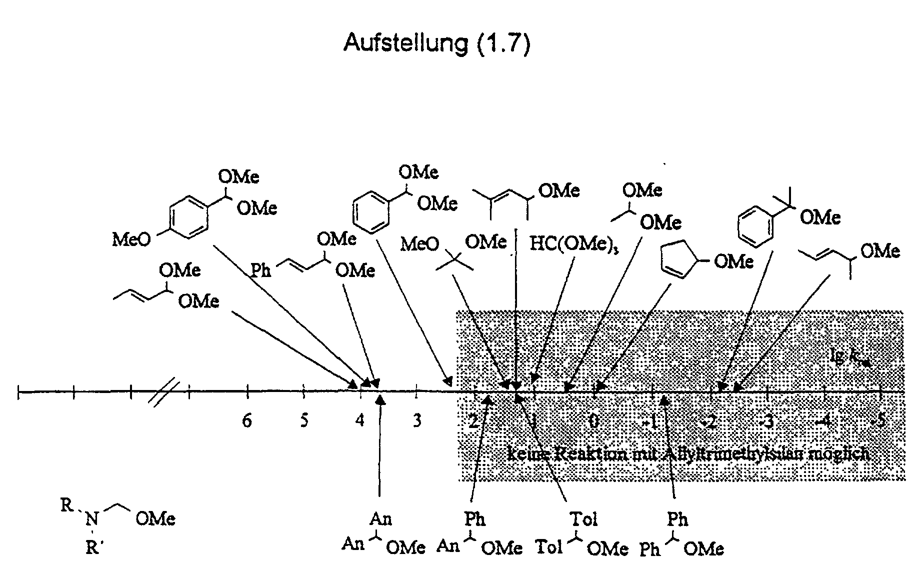

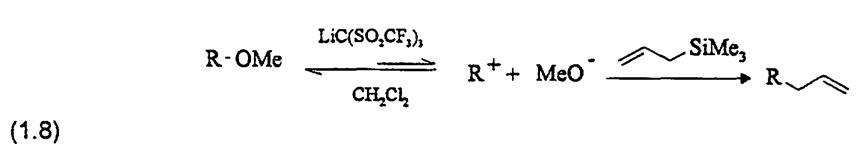

Nach dem vorstehenden Reaktionsschema (1.8) sind Umsetzungen von Acetalen bzw. Methylethern möglich, deren relative Reaktivitätskonstante kᵣₑᵢ ≥ der von Benzaldehyddimethylacetal ist.

Die nachfolgende Aufstellung (1.9) stellt ausgewählte Methylether und Acetale dar, die als mögliche Reaktionspartner von Allyltrimethylsilan oder reaktiveren Nucleophilen (N ≥ 1,62) in einer Suspension aus Mg(C(SO₂CF₃)₃)₂ in Dichlormethan in Frage kommen.

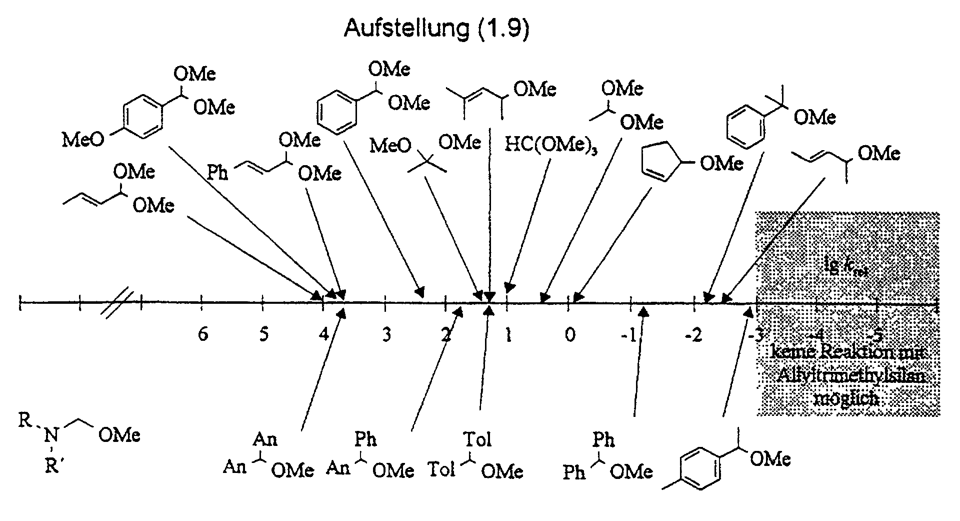

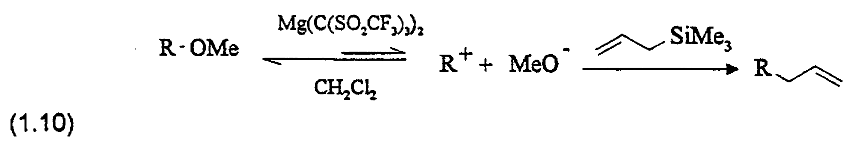

Gemäß dem vorstehenden Reaktionsschema (1.10) können Methylether oder Acetale mit Nucleophilen, deren N ≥ 1,62 ist, umgesetzt werden, sofern ihre relative Reaktivitätskonstante kᵣₑₗ gleich oder größer der von 1-Tolyl-1-methoxyethan ist.

*In den vorstehenden Aufstellungen haben die Abkürzungen folgende Bedeutung:*
- An: 4-Methoxyphenyl-Rest
- Ph: Phenyl-Rest
- Tol: 4-Methylphenyl-Rest
- Me: Methyl-Rest

Die erfindungsgemäß zum Einsatz kommenden Lewis-Säure-Katalysatoren können auch Reaktionen von Aldehyden, deren Reaktivität ≥ der von Benzaldehyd gegenüber Nucleophilen mit einer Nucleophilie N ≥ 5, wie z. B. gegenüber 1-Phenyl-1-trimethylsiloxy-ethen, 1-Trimethylsiloxycyclopenten oder 1-Methoxy-1-trimethylsiloxy-2-methyl-1-propen induzieren. Als Aldehyde sind insbesondere Benzaldehyd oder Methoxybenzaldehyd geeignet. Vorzugsweise werden hierfür Suspensionen von Li-Salzen oder Mg-Salzen der Formel (1) als Lewis-Säure Katalysatoren verwendet.

Vorzugsweise wird dieses Lewis-Säure Katalysatorsystem auch zur Induktion der Umsetzung von Ketonen, deren Reaktivität gleich oder größer als die von Acetophenon gegenüber Nucleophilen mit einer Nucleophilie N ≥ 9, wie z. B. Methoxy-1-trimethylsiloxy-2-methyl-1-propen ist, verwendet.

Ebenso bevorzugt ist der Einsatz von Li- oder Mg-Salzen der Formel (1) als Lewis-Säure Katalysatoren, vorzugsweise ohne Zusatz eines Lösungsmittels, zur Katalyse der Umsetzung von Carbonsäurehalogeniden, insbesondere Carbonsäurechloriden oder -bromiden, deren Reaktivität gleich oder größer als die von Benzoylchlorid gegenüber Nucleophilen mit einer Nucleophilie N ≥ 9, wie z. B. 1-Methoxy-2-methyl-1-trimethylsiloxy-propan ist.

Dies gilt auch für die Umsetzung von Carbonsäureanhydriden, deren Reaktivität gleich oder größer als die von Acetanhydrid gegenüber Nucleophilen mit einer Nucleophilie N ≥ -2ist.

Li- und Mg-Salze der Formel (1) induzieren auch die [4+2]-Cycloaddition beliebiger Diene, wie z. B. 2,3-Dimethyl-1,3-butadien oder Cyclopentadien. Die mit den Dienen umzusetzenden Olefine, tragen bevorzugt elektronenziehende Substituenten. Beispiele sind Methylvinylketon oder Maleinsäureanhydrid. Die Cycloadditionen verlaufen i. a. bereits bei Raumtemperatur und sind gegenüber den unkatalysieren Reaktionen deutlich beschleunigt, i. a. um einen Faktor von etwa 1,5. Als zusätzlicher Effekt wird die Bildung des endo-Produkts bevorzugt. Für [4+2]-Cycloadditionen ist die Verwendung von Verbindungen der Formel (1) als Suspension in Dichlormethan bevorzugt, wobei die Konzentration 2-20 mol%, vorzugsweise 5-15 mol% bezogen auf das zu aktivierende Dienophil beträgt.

Die Reaktionen werden i. a. bei einer Temperatur von 0 bis 60°C, bevorzugt bei 10 bis 30°C, unter Inertgas durchgeführt.

Bevorzugterweise eignet sich Tris(trifluoromethylsulfonyl)methan in einer Konzentration von 0,5-10 mol%, vorzugsweise 1-5 mol%, bezogen auf das elektrophile Carbonsäurehalogenid, vorzugsweise Carbonsäurechlorid, wie z. B. Acetylchlorid oder Benzoylchlorid zur Katalyse von Friedel Crafts-Acylierungen. Dabei sollen vorzugsweise Reaktionstemperaturen von 60-105°C, bevorzugt 80-110°C eingehalten werden.

Dies gilt auch für eine entsprechende intramolekulare Friedel-Crafts-Acylierung.

Die Reaktionszeiten betragen im allgemeinen 2 bis 250 Stunden, bevorzugt 3 bis 72 Stunden und besonders bevorzugt 12 bis 48 Stunden.

Die Edukte, Elektrophil und Nucleophil, werden entweder als Lösung im Lösungsmittel oder Suspensionsmittel der Katalysatorverbindung der Formel (1), wie z. B. Diethylether oder Dichlormethan, oder, falls sie bei der Reaktionstemperatur flüssig sind, in Substanz eingesetzt.

Die für die erfindungsgemäßen Reaktionen verwendeten Lösungsmittel bzw. Suspensionsmittel sind jeweils wasserfrei; sie werden auf bekannte Weise absolutiert.

Der Verlauf der Reaktion kann jeweils mit GC oder HPLC verfolgt werden.

Nach Beendigung der Reaktion wird das Reaktionsgemisch auf übliche Art aufgearbeitet, z. B. durch Hydrolyse mit Wasser und Extraktion bei Einsatz koordinierender Lösungsmittel oder aber durch Abfiltrieren bei Einsatz nicht koordinierender Lösungsmittel. Das Rohprodukt kann z. B. durch Chromatographie, Umkristallisation oder Destillation gereinigt werden.

Die Verbindung der Formel (1) wird entweder durch Abfiltrieren der Reaktionsmischung oder durch Eindampfen der nach der Hydrolyse der Reaktionsmischung anfallenden wäßrigen Phase zurückgewonnen. Die so zurückerhaltene Verbindung (1) kann auf übliche Art, z. B. durch Suspendieren in einem geeigneten Lösungsmittel wie z. B. Dichlormethan/Pentan (1/1 v/v) und anschließendes Absaugen gereinigt werden. Sie wird anschließend auf übliche Weise bis zur Gewichtskonstanz getrocknet, z. B. bei 120-170°C/0,01 bis 0,1 mbar, bevor sie wiedereingesetzt wird.

Weitere Reaktionsparameter sind den allgemeinen Arbeitsvorschriften für homogene bzw. heterogene Synthesen, die mit Hilfe von den erfindungsgemäßen Katalysatoren der Formel (1) induziert werden, zu entnehmen, wobei diese Angaben auf alle erfindungsgemäß zum Einsatz kommenden Verbindungen der Formel (1) bei entsprechender homogener oder heterogener Synthese angewendet werden können.

Ein weiterer Gegenstand der Erfindung ist die erfindungsgemäß zum Einsatz kommende Mg-Verbindung der Formel (1).

Dieses Mg-tris[(trifluormethyl)sulfonyl]methanid kann durch Umsetzung von Magnesiumcarbonat mit Tris(trifluormethyl)sulfonyl)-methan vorzugsweise in stöchiometrischen Mengen erhalten werden. Als Lösungsvermittler wurde soviel Wasser zugesetzt, bis eine leichte Gasentwicklung einsetzte und die Feststoffe in Lösung gingen. Man erhält eine stark viskose farblose Lösung. Aus dieser Lösung kann das Mg-Salz der Formel (1) durch Trocknung gewonnen werden. Da das Salz stark hygroskopisch ist, muß es unter Schutzgas aufbewahrt werden.

### Beispiel C:

### Allgemeine Arbeitsvorschrift 1 für homogene Synthesen in etherischen

### LiC(SO₂CF₃)₃-Lösungen

Zu 5 bis 10 ml einer 0.03 m Lösung von LiC(SO₂CF₃)₃ in abs. Diethylether werden unter Inertgasatmosphäre (Stickstoff) die Edukte (jeweils 50 mmol), entweder als Flüssigkeiten oder als Lösungen in Diethylether getropft. Dabei beträgt die Konzentration des Li-Salzes 1 bis10 mol% bezogen auf das Elektrophil. Es wird bis zur angegebenen Reaktionszeit bei 20 °C gerührt. Zur Entfernung des Lithiumsalzes aus der etherischen Phase wird unter heftigem Rühren 10 ml Wasser zugegeben. Anschließend werden die Phasen getrennt und die wäßrige Phase wird dreimal mit je 10 mL Dichlormethan extrahiert. Die vereinigten organischen Phasen werden über Magnesiumsulfat getrocknet, filtriert und die Lösungsmittel werden i. Vak. entfernt. Das verbleibende Rohprodukt wird durch Umkristallisation oder Destillation gereinigt.

Zur Wiedergewinnung des LiC(SO₂CF₃)₃ wird die wäßrige Phase i. Vak. eingeengt und das kristallisierende Salz abgesaugt. Zur Entfernung eventuell anhaftender organischer Substanzen wird das LiC(SO₂CF₃)₃ in einem Gemisch aus Dichlormethan und Pentan (1/1 v/v) suspendiert und anschließend abgesaugt. Das so erhaltene Salz wird bis zur Gewichtskonstanz bei 150 °C / 0.01 mbar getrocknet.

### Allgemeine Arbeitsvorschrift 2 für heterogene Synthesen mit LiC(SO₂CF₃)₃ bzw. Mg(C(SO₂CF₃)₃)₂ in CH₂Cl₂

Zu einer Suspension von 0.05 bis 0.50 g des Methanids in 20 bis 30 ml trockenem Dichlormethan werden unter Inertgasatmosphäre (Stickstoff) die Edukte (jeweils 50 mmol), entweder als Flüssigkeiten oder als Lösungen in Dichlormethan, zum heterogenen Salz-Dichlormethan-Gemisch getropft. Dabei beträgt die Konzentration des Li-Salzes 1 bis 10 mol% bezogen auf das Elektrophil. Es wird bis zur angegeben Zeit bei 20 °C gerührt. Nach Filtration des Reaktionsgemisches wird das Dichlormethan i. Vak. entfernt. Das verbleibende Rohprodukt wird durch Umkristallisation oder Destillation gereinigt. Das im Filtrat befindliche Methanid läßt sich für weitere Synthesen nach Umkristallisation wiederverwenden.

### Allgemeine Arbeitsvorschrift 3 für homogene Synthesen mit HC(SO₂CF₃)₃ ohne Lösungsmittel

Zu einem Gemisch der Edukte (jeweils 50 mmol) wird unter Inertgasatmosphäre (Stickstoff) Tris(trifluoromethylsulfonyl)methan (ca. 0.1 mmol) zugegeben. Dabei beträgt die Konzentration der HC(SO₂CF₃)₃ 0,002-0,01 mmol/mmol Elektrophil.

Das Reaktionsgemisch wird bis zur angegebenen Zeit bei 20 °C bzw. 80 bis 110 °C gerührt. Nach Abkühlung auf Raumtemperatur werden 15 mL Wasser und 25 ml Diethylether zugegeben, die Phasen getrennt und die wäßrige Phase drei mal mit je 10 ml Diethyether extrahiert. Die vereinigten Pasen werden über MgSO₄ getrocknet, filtriert und das Lösungsmittel wird i. Vak. entfernt. Das verbleibende Rohprodukt wird durch Umkristallisation oder Destillation gereinigt. HC(CO₂CF₃)₃ wird zurückgewonnen wie in der allgemeinen Arbeitsvorschrift 1 beschrieben.

Die Ergebnisse der nach den vorstehenden Arbeitsvorschriften durchgeführten Reaktionen sind in den folgenden Tabellen und Schemata zusammengefaßt, wobei folgende Abkürzungen verwendet wurden:
- An: 4-Methoxyphenyl-Rest
- Ad: Adenyl-Rest
- Tol: 4-Methylphenyl-Rest

### Beispiele 1-8

Gemäß Arbeitsvorschrift 1 bzw. 2 wurden mit Lithium-tris(trifluoromethylsulfonyl)methanid induzierte Synthesen von Alkylchloriden mit Allyltrimethylsilan durchgeführt. Die Resultate der Umsetzung sind in Tabelle 1 zusammengefaßt

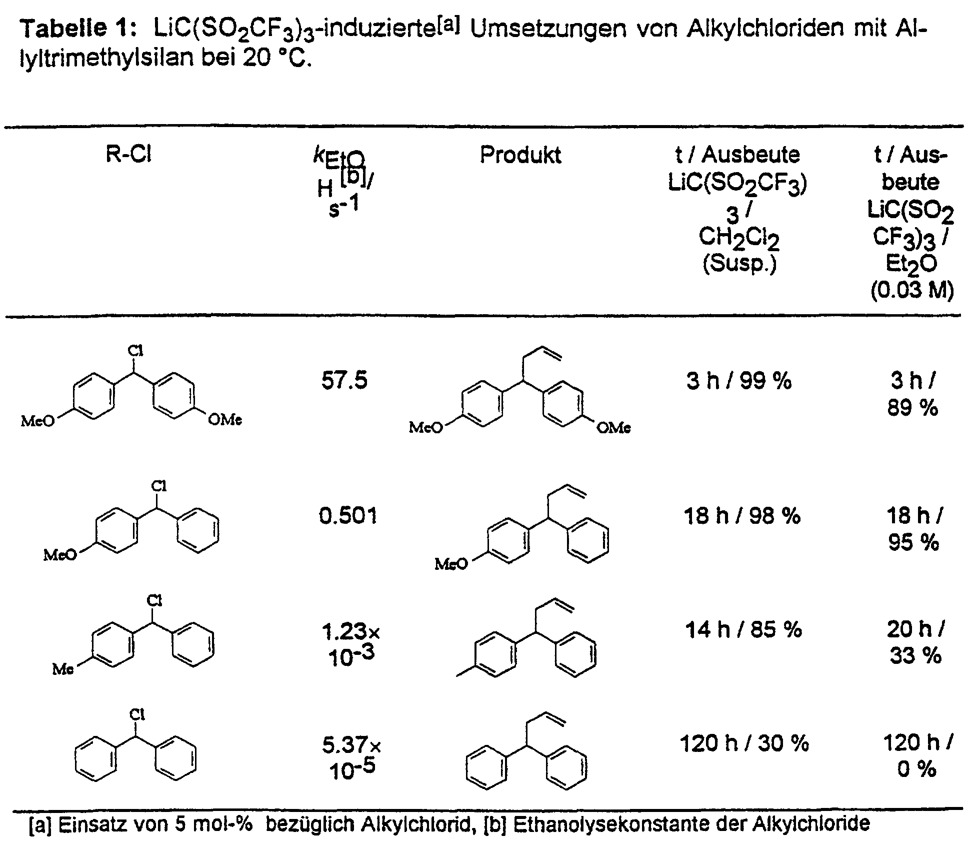

### Beispiele 9-12

Gemäß Arbeitsvorschrift 2 wurden mit Lithium-tris(trifluoromethylsulfonyl)methanid induzierte Synthesen von Acetalen und Methylethern mit Allyltrimethylsilan durchgeführt.
Die Resultate sind in Tabelle 2 zusammengefaßt

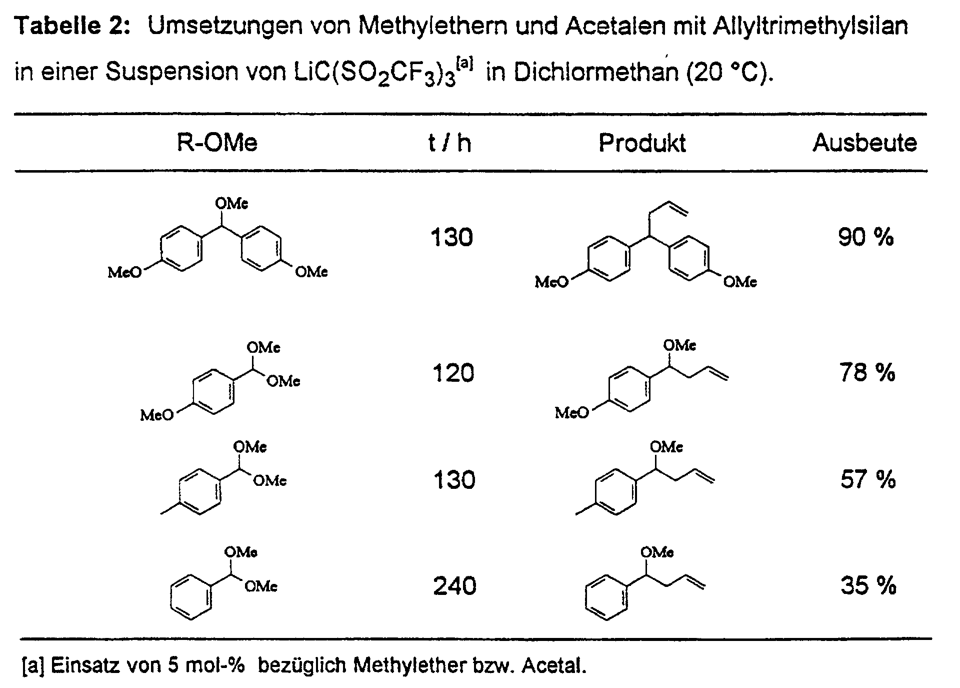

Während sich Bis-(4-methoxyphenyl)methylchlorid in einer Suspension von Lithium-tris(trifluoromethylsulfonyl)methanid in Dichlormethan innerhalb von drei Stunden mit Allyltrimethylsilan quantitativ zum allylierten Produkt umsetzen läßt (Tabelle 1), liefert der analoge Methylether Bis-(4-methoxyphenyl)methoxymethan mit Allyltrimethylsilan erst innerhalb von 120 Stunden in 90 % Ausbeute das allylierte Produkt Ausbeute (Tabelle 2). Die Lithium-tris(trifluoromethylsulfonyl)methanid induzierte Umsetzung von Anisaldehyddimethylacetal mit Allyltrimethylsilan liefert innerhalb von 130 Stunden den entsprechenden Homoallylalkohol in 78 % Ausbeute. Das weniger reaktive Tolylaldehyddimethylacetal bzw. Benzaldehyddimethylacetal reagieren innerhalb von 130 bzw. 240 Stunden in deutlich schlechteren Ausbeuten zu den entsprechenden Homoallylethem.

### Beispiele 13-14

Gemäß Arbeitsvorschrift 2 wurden mit Lithium-tris(trifluoromethylsulfonyl)methanid induzierte Synthesen von N,O-Acetalen mit 1 -Methoxy-2-methyl-1-trimethylsiloxy-propan durchgeführt.

Das lonisationsvermögen einer Suspension von Lithium-tris(trifluoromethylsulfonyl)methanid in Dichlormethan ist ausreichend groß um aus *N*,*O*-Acetalen Iminiumionen zu generieren. Die durch eine Suspension von Lithium-tris(trifluoromethylsulfonyl)methanid in Dichlormethan induzierte Umsetzung der *N*,*O*-Acetale mit 1-Methoxy-2-methyl-1-trimethylsiloxy-propen liefert die entsprechenden Aminoester in sehr guten Ausbeuten (Tabelle 3).

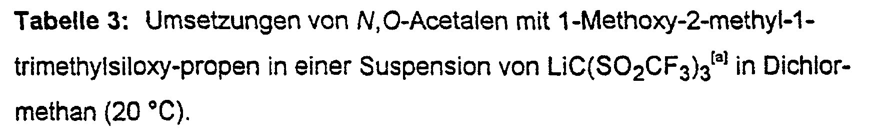

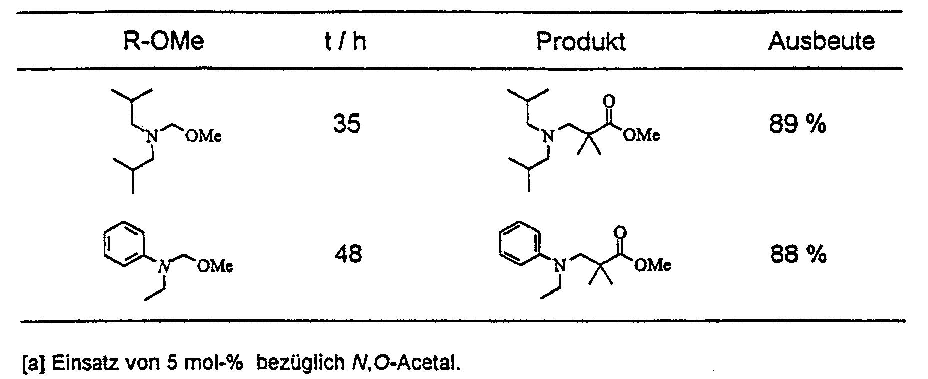

### Beispiele 15-17

Gemäß der allgemeinen Arbeitsvorschrift 2 wurden mit Lithium-tris(trifluoromethylsulfonyl)methanid induzierte Synthesen von Benzaldehyd mit den in der Tabelle 4 angegebenen Nucelophilen durchgeführt

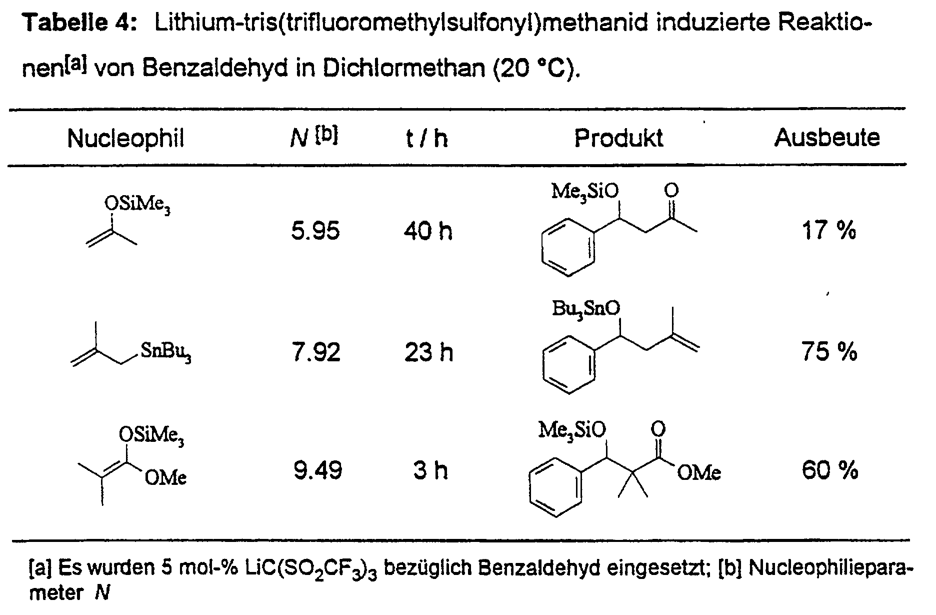

### b

Gemäß der allgemeinen Arbeitsvorschrift 2 wurde eine Umsetzung von Acetophenon mit 1-Methoxy-2-methyl-1-trimethylsiloxy-propen nach folgendem Reaktionsschema durchgeführt

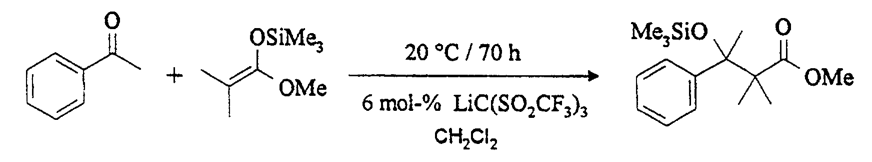

Die Ausbeute des Produktes betrug 44 %

### Beispiel 19

### Umsetzung von Acetanhydrid

Erhitzt man ein Gemisch von Acetanhydrid und Anisol (N = -1.56) in Anwesenheit von 6 mol-% Lithium-tris(trifluoromethylsulfonyl)methanid 48 Stunden lang ohne den Zusatz eines Lösungsmittels auf 120 °C, isoliert man das Acylierungsprodukt in 41 % Ausbeute.

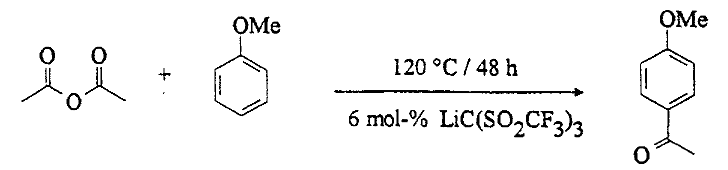

### Beispiel 20

### Umsetzung von Benzoylchlorid

Unter analogen Reaktionsbedingungen wie in Beispiel 19 gelingt die Umsetzung von Benzoylchlorid mit dem deutlich nucleophileren 1-Methoxy-2-methyl-1-trimethylsiloxy-propen (*N* = 9.49). Man erhält das Acylierungsprodukt innerhalb von 24 Stunden in 72 % Ausbeute.

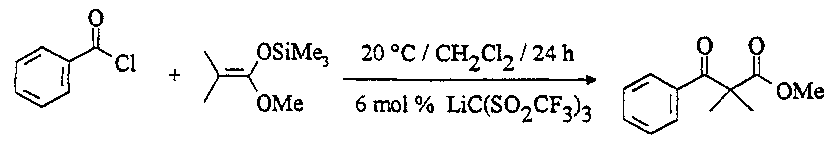

### Beispiele 21-23

### Lithium-tris(trifluoromethylsulfonyl)methanid induzierte [4+2]-Cycloadditionen

### Cycloaddition mit 2,3-Dimethyl-1,3-butadien

In Gegenwart von 15 mol-% Lithium-tris(trifluoromethylsulfonyl)methanid in

Dichlormethan erhält man innerhalb von 60 Stunden aus 2,3-Dimethyl-1,3-butadien und Methylvinylketon das [4+2]-Cycloaddukt in 50 % Ausbeute. Der aktivierende Effekt von Lithium-tris(trifluoromethylsulfonyl)methanid auf diese Cycloaddition ist in Diethylether wegen koordinierender Wechselwirkungen zwischen dem Lewis-aciden Lithiumkation und dem Sauerstoff des Ethers deutlich schwächer ausgeprägt. Man erhält das Cycloaddukt nach 20 Stunden Reaktionszeit in nur 2 % Ausbeute (Tabelle 5). Zum Vergleich: Die unkatalysierte Reaktion dauert bei quantitativen Umsatz der Edukte bei 20 °C 4000 Stunden (167 Tage).

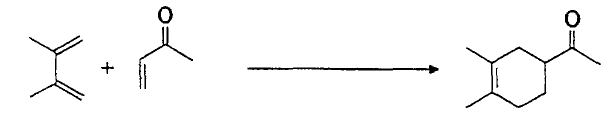

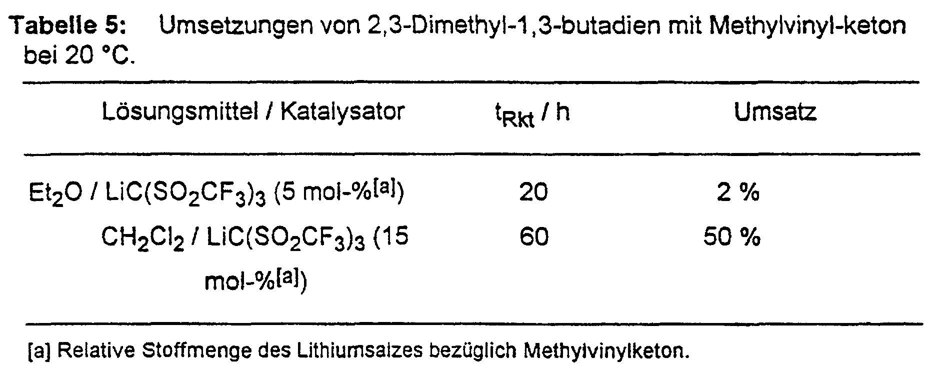

### Cycloaddition mit Cyclopentadien gemäß dem nachfolgenden Reaktionsschema

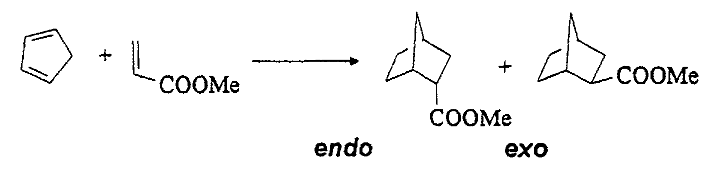

Die Reaktion von Cyclopentadien mit Methylacrylat gemäß den vorstehenden Bedingungen wird durch Zugabe von 2 mol-% Lithium-tris(trifluoromethylsulfonyl)methanid bezüglich Methylacrylat zum Lösungsmittel Dichlormethan um den Faktor 1.5 beschleunigt . Das *endo* / *exo -* Verhältnis beträgt

### Beispiele 24-27

### A) Herstellung von Magnesiumtris((trifluormethyl)sulfonyl)methanid

Man vereinigt 0,8153 g, (1,979 mmol) Tris(trifluormethylsulfonyl)methan und 0,0962 g (0,198 mmol) basisches Magnesiumcarbonat (4 MgCO₃-Mg(OH)₂-5H₂O) und vermengt dies mit 0,3 ml Wasser, wobei CO₂ freigesetzt wird. Die hoch viskose Flüssigkeit läßt man 3 h bei Raumtemperatur rühren. Das nach Entfernen des Lösungsmittel i. Vak. erhaltene farblose kristalline Pulver läßt man 2 d bei 50°C (1,2 x 10⁻² mbar) trocknen. Man erhält das Magnesiumsalz als farblose stark hygroskopische Kristallite.

Ausbeute: 96% (0,185 g) mit Schmelzpunkt 141-143°C.

### B) Gemäß Arbeitsvorschrift 2 wurden mit den nach A) hergestellten Magnesium-tris(trifluoromethylsulfonyl)methanid induzierte Synthesen von Alkylchloriden mit Allyltrimethylsilan durchgeführt. Die Resultate sind Tabelle 6 zu entnehmen.

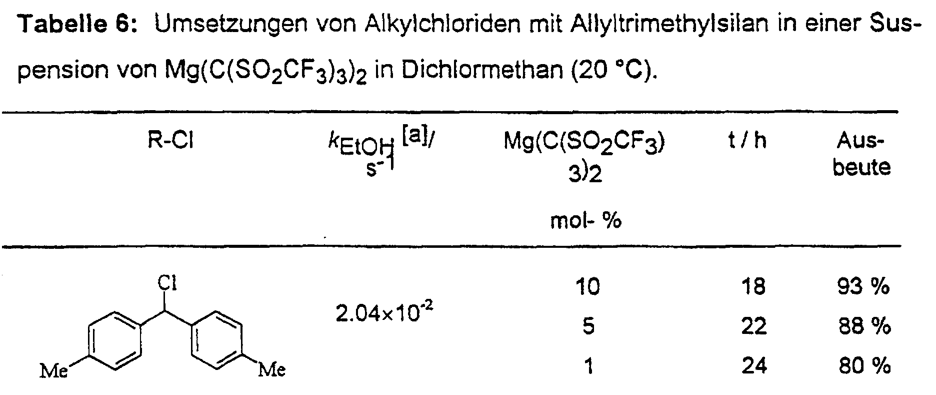

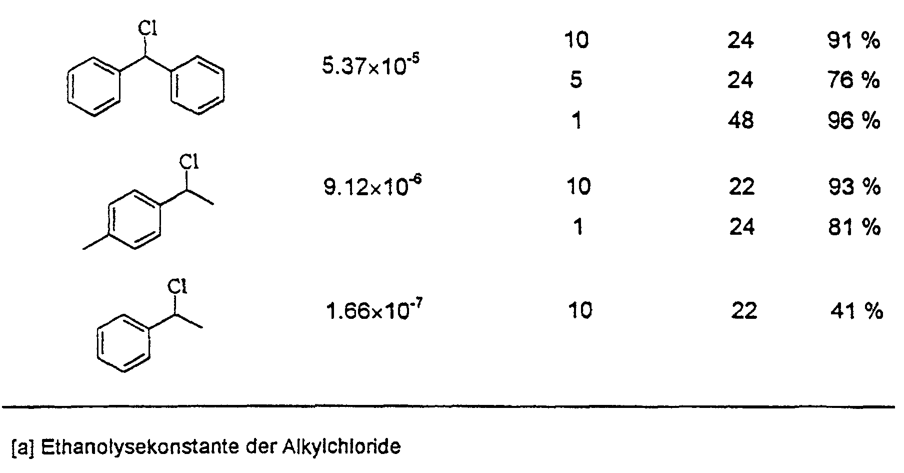

### Beispiele 28-29

Gemäß Arbeitsvorschrift 2 wurden unter Einsatz des nach Beispiel 24-27 A) hergestellten Magnesium-tris(trifluoromethylsulfonyl)methanid Umsetzungen von Methylethern mit Allyltrimethylsilan durchgeführt. Die Resultate sind in Tabelle 7 zusammengefaßt.

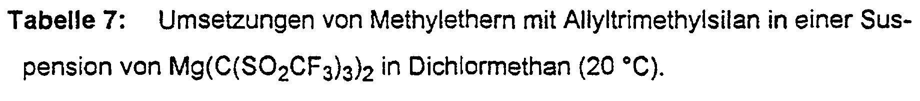

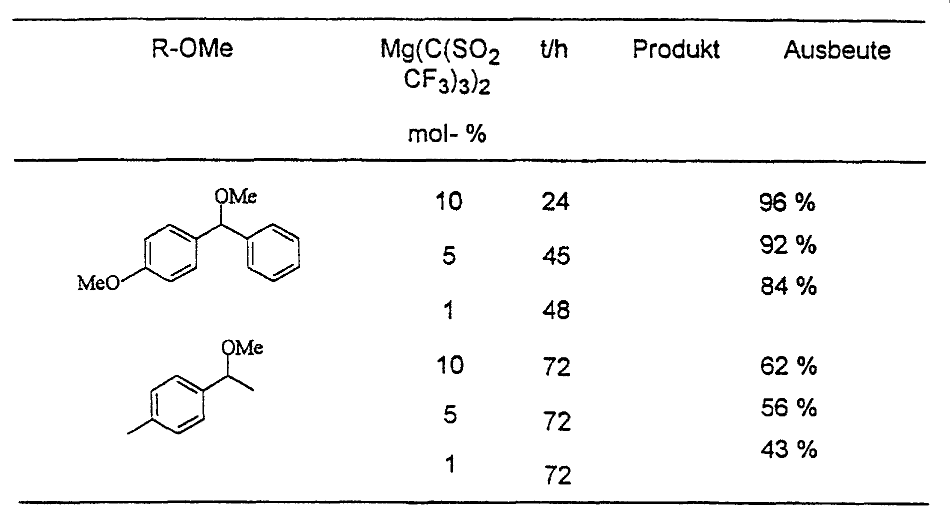

### Beispiele 30-31

Gemäß Arbeitsvorschrift 3 wurden mit Tris(trifluoromethylsulfonyl)-methan induzierte Friedel-Crafts-Acylierungen nach dem nachstehenden Reaktionsschema mit den angeführten Benzolderivaten durchgeführt. Die Resultate sind Tabelle 8 zu entnehmen.

### Synthesen von Acetylchlorid mit Benzolderivaten

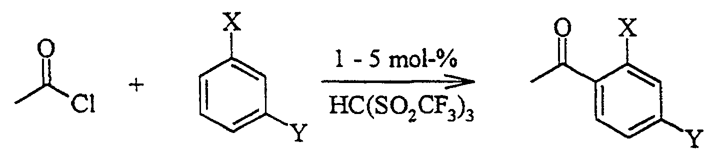

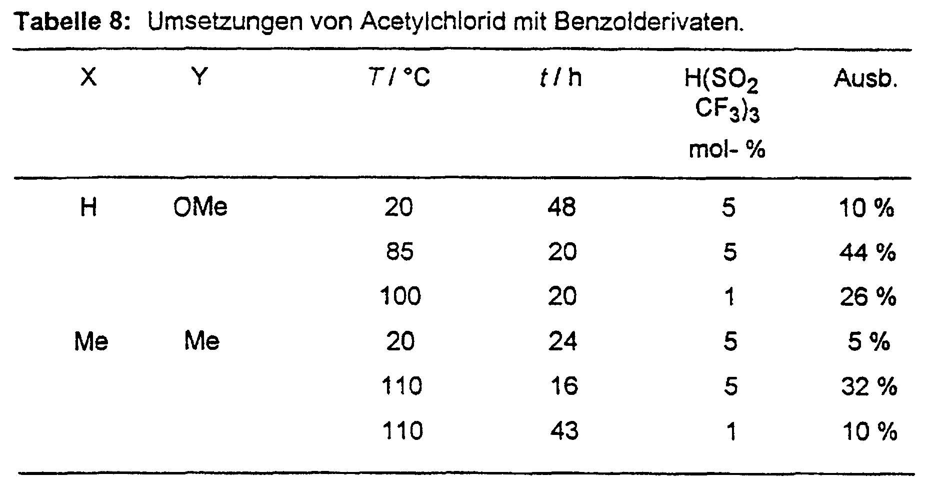

### Beispiele 32-34

Gemäß Arbeitsvorschrift 3 wurden mit Tris(trifluoromethylsulfonyl)methan induzierte Umsetzungen von Benzoylchlorid mit Benzolderivaten gemäß Tabelle 9 durchgeführt. Die Resultate sind Tabelle 9 zu entnehmen.

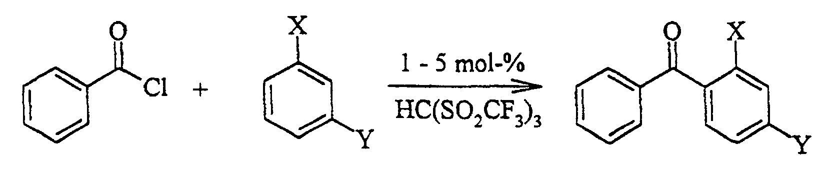

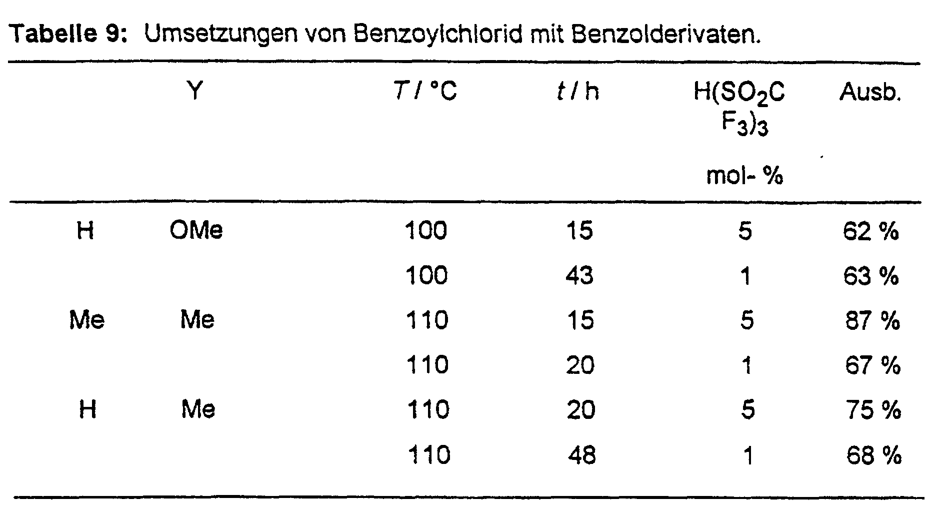

### Beispiele 35-36

### Intramolekulare Friedel-Crafts-Acylierungen

Gemäß Arbeitsvorschrift 3 wurden mit Tris(trifluoromethylsulfonyl)-methan als Katalysator die intramolekulare Friedel-Crafts-Acylierung von *o* Benzylbenzoesäure bzw. *o*-Benzoylbenzoesäure zu Anthron bzw. Anthrachinon durchgeführt. Die Ausbeute der Produkte lag bei 45 %.

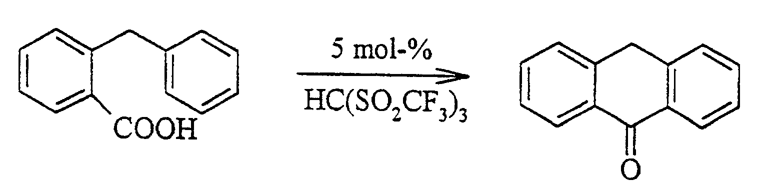

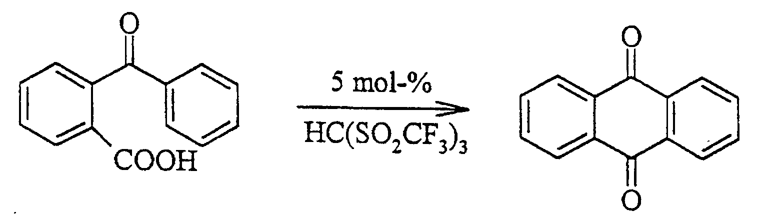

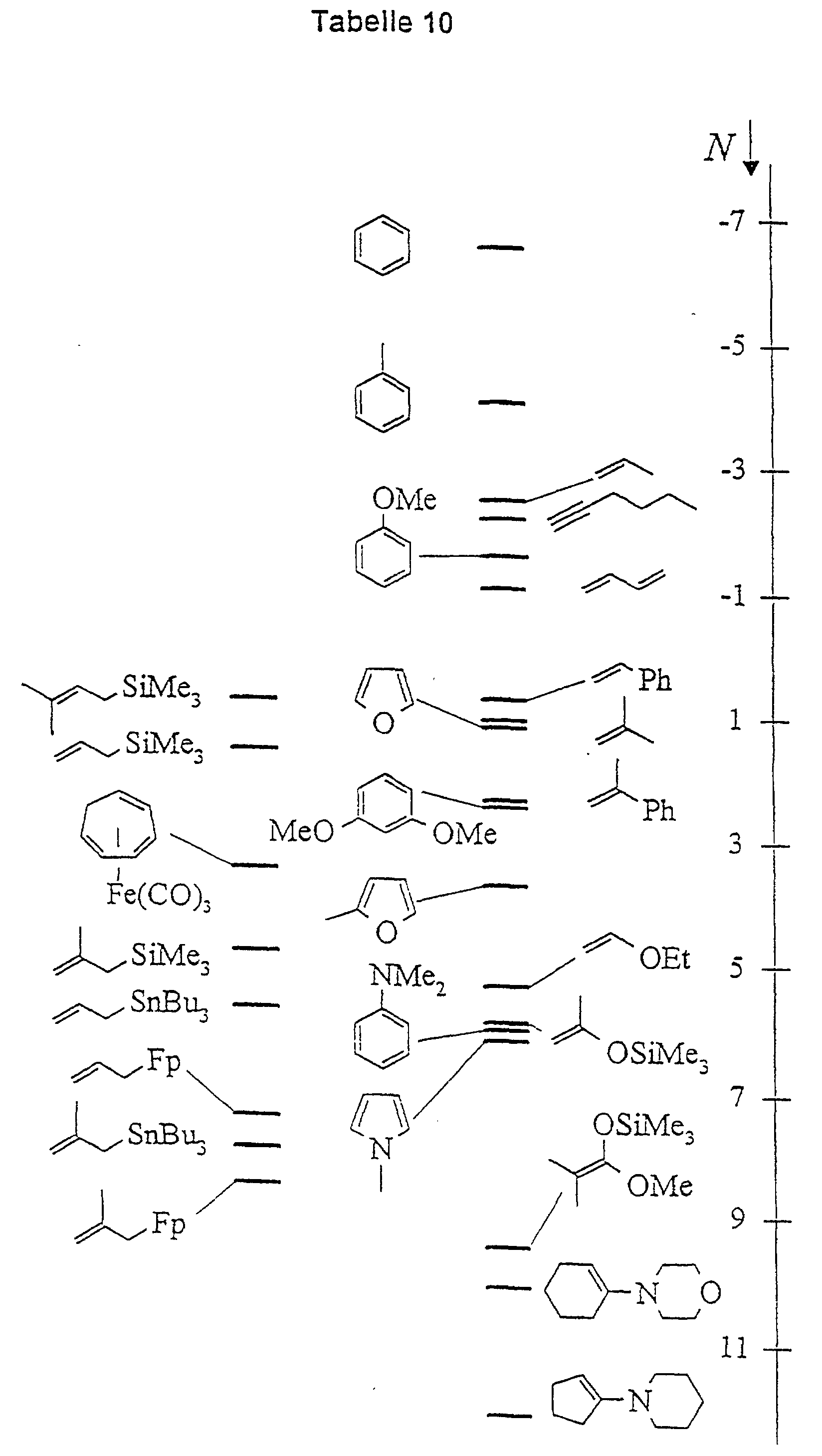

## Patentansprüche

1. Verwendung von wenigstens einer Verbindung der Formel (1)
M^{+x}(C(SO₂CF₃)₃)ₓ (1)
worin x 1 oder 2 bedeutet,
M für ein Wasserstoff- oder Alkalimetallatom steht, wenn x 1 bedeutet, bzw. für ein Erdalkalimetallatom steht, wenn x 2 bedeutet, als Katalysator bei C-C verknüpfenden Synthesen.

2. Verwendung nach Anspruch 1 bei der Umsetzung von durch Lewis-Säuren aktivierbaren Elektrophilen, insbesondere Akylhalogeniden, Dialkylacetalen, Alkylethern, N,O-Acetalen, Ketonen, Aldehyden, Carbonsäurehalogeniden oder -anhydriden mit Nucleophilen oder bei [4+2]-Cycloadditionen.

3. Verwendung nach Anspruch 1 oder 2, dadurch gekennzeichnet, daß M Wasserstoff, Lithium, Natrium, Kalium, Magnesium oder Calcium, insbesondere Wasserstoff, Lithium oder Magnesium bedeutet

4. Verwendung nach Anspruch 1 oder 2, dadurch gekennzeichnet, daß die Verbindungen der Formel (1) als homogene Lösung in koordinierenden, organischen Lösungsmitteln, vorzugsweise in Diethylether, eingesetzt werden.

5. Verwendung nach Anspruch 1 oder 2, dadurch gekennzeichnet, daß die Verbindungen der Formel (1) als Suspension in nicht koordinierenden, organischen Lösungsmitteln, vorzugsweise in Dichlormethan, eingesetzt werden.

6. Verwendung nach Anspruch 1, dadurch gekennzeichnet, daß Lithium-tris(trifluormethylsulfonyl)methanid, suspendiert in einem nicht koordinierenden Lösungsmittel, vorzugsweise in Dichlormethan, als Lewis-Säure Katalysator bei der Umsetzung von Alkylhalogeniden, vorzugsweise Chloriden oder Bromiden, mit einer Ethanolysegeschwindigkeit.*k*_{EtOH}(25°C) > 1x10⁻⁵ s⁻¹ mit Nucleophilen, deren Nucleophilie N ≥ 1 ist eingesetzt wird.

7. Verwendung nach Anspruch 1, dadurch gekennzeichnet, daß Lithium-tris(trifluormethylsulfonyl)methanid, gelöst in einem koordinierenden, organischen Lösungsmittel, vorzugsweise in Diethylether, als Lewis-Säure Katalysator bei der Reaktion von Alkylhalogeniden, vorzugsweise Alkylchloriden oder Alkylbromiden, mit einer Ethanolysegeschwindigkeit *k*_{EtOH}(25°C) > 1x10⁻³ s⁻¹ mit Nucleophilen, deren Nucleophilie N ≥1 ist, eingesetzt wird.

8. Verwendung nach Anspruch1, dadurch gekennzeichnet, daß Magnesium-tris(trifluormethylsulfonyl)methanid, suspendiert in einem nicht koordinierenden Lösungsmittel, vorzugsweise in Dichlormethan, bei Reaktion von Alkylhalogeniden, vorzugsweise Alkylchloriden oder Bromiden mit einer Ethanolysegeschwindigkeit k _{EtOH} (25°C) > 1x10⁻⁷ s⁻¹ mit Nucleophilen, deren Nucleophilie N ≥ 1 ist, eingesetzt wird.

9. Verwendung nach Anspruch1, dadurch gekennzeichnet, daß Lithium-tris(trifluormethylsulfonyl)methanid, als Suspension in nicht koordinierenden Lösungsmitteln, vorzugsweise Dichlormethan, eingesetzt wird und die Reaktion von Alkylethern oder Alkylacetalen mit einer relativen Reaktivität kᵣₑₗ, die größer ist als die von Benzaldehyddimethylacetal ist, mit Nucleophilen, deren Nucleophilie N ≥ 1 ist, katalysiert wird.

10. Verwendung nach Anspruch1, dadurch gekennzeichnet, daß Magnesium-tris(trifluormethylsulfonyl)methanid als Suspension in einem nicht koordinierendem Lösungsmittel, vorzugsweise in Dichlormethan, eingesetzt wird und die Reaktion von Alkylethern oder Alkylacetalen mit einer relativen Reaktivität kᵣₑₗ, die größer ist als die von 1-Tolyl-1-methoxy-ethan ist, mit Nucleophilen, deren Nucleophilie N ≥ 1 ist, katalysiert wird.

11. Verwendung nach Anspruch 1, dadurch gekennzeichnet, daß Lithium- oder Mg-tris(trifluormethylsulfonyl)methanid als Suspension in nicht koordinierenden Lösungsmitteln, vorzugsweise in Dichlormethan, eingesetzt wird und die Reaktion von Aldehyden mit einer Reaktivität, die mindestens gleich oder größer derjenigen von Benzaldehyd ist, mit Nucleophilen, deren Nucleophilie N ≥ 5 ist, katalysiert wird.

12. Verwendung nach Anspruch 1, dadurch gekennzeichnet, daß Lithium- oder Mg-tris(trifluormethylsulfonyl)methanid als Suspension in nicht koordinierenden Lösungsmitteln, vorzugsweise in Dichlormethan, eingesetzt wird und die Reaktion von Ketonen mit einer Reaktivität, die mindestens gleich oder größer derjenigen von Acetophenon ist mit Nucleophilen, deren Nucleophilie N ≥ 9 ist, katalysiert wird.

13. Verwendung nach Anspruch 1, dadurch gekennzeichnet, daß Lithium- oder Mg-tris(trifluormethylsulfonyl)methanid eingesetzt wird und die Umsetzung von Carbonsäurehalogeniden, insbesondere Carbonsäurechloriden oder Bromiden, deren Reaktivität gleich oder größer als die von Benzoylchlorid istgegenüber Nucleophilen mit einer Nucleophilie N ≥ 9 katalysiert wird.

14. Verwendung nach Anspruch 1, dadurch gekennzeichnet, daß Lithium- oder Mg-tris(trifluormethylsulfonyl)methanid eingesetzt wird und die Umsetzung von Carbonsäureanhydriden, deren Reaktivität gleich oder größer als die von Acetanhydrid gegenüber Nucleophilen mit einer Nucleophilie N ≥ -2 katalysiert wird.

15. Verwendung nach Anspruch 1, dadurch gekennzeichnet, daß Tris(trifluormethylsulfonyl)methan eingesetzt wird und Friedel-Crafts-Acylierungen katalysiert werden.

## Claims

1. Use of at least one compound of the formula (1)
M^{+x}(C(SO₂CF₃)₃)ₓ (1)
where x is 1 or 2,
M is a hydrogen atom or alkali metal atom when x is 1, or is an alkaline earth metal atom when x is 2, as catalyst in carbon-carbon bond-forming syntheses.

2. Use according to Claim 1 in the reaction of Lewis-acid-activatable electrophiles, in particular alkyl halides, dialkylacetals, alkyl ethers, N,O-acetals, ketones, aldehydes, acyl halides or carboxylic anhydrides with nucleophiles or in [4+2]-cycloadditions.

3. Use according to Claim 1 or 2, characterized in that M is hydrogen, lithium, sodium, potassium, magnesium or calcium, in particular hydrogen, lithium or magnesium.

4. Use according to Claim 1 or 2, characterized in that the compounds of the formula (1) are used as a homogeneous solution in coordinating, organic solvents, preferably in diethyl ether.

5. Use according to Claim 1 or 2, characterized in that the compounds of the formula (1) are used as a suspension in non-coordinating organic solvents, preferably in dichloromethane.

6. Use according to Claim 1, characterized in that lithium tris (trifluoromethylsulfonyl)methanide, suspended in a non-coordinating solvent, preferably in dichloromethane, is used as Lewis acid catalyst in the reaction of alkyl halides, preferably chlorides or bromides, having an ethanolysis rate *k*_{EtOH} (25°C) > 1 × 10⁻⁵ s⁻¹ with nucleophiles having a nucleophilicity N ≥ 1.

7. Use according to Claim 1, characterized in that lithium tris (trifluoromethylsulfonyl)methanide, dissolved in a coordinating, organic solvent, preferably in diethyl ether, is used as Lewis acid catalyst in the reaction of alkyl halides, preferably alkyl chlorides or alkyl bromides, having an ethanolysis rate *k*_{EtOH} (25°C) > 1 × 10⁻³ s⁻¹ with nucleophiles having a nucleophilicity N ≥ 1.

8. Use according to Claim 1, characterized in that magnesium tris (trifluoromethylsulfonyl)methanide, suspended in a non-coordinating solvent, preferably in dichloromethane, is used in the reaction of alkyl halides, preferably alkyl chlorides or alkyl bromides having an ethanolysis rate *k*_{EtOH} (25°C) > 1 x 10⁻⁷ S⁻¹ with nucleophiles having a nucleophilicity N ≥ 1.

9. Use according to Claim 1, characterized in that lithium tris (trifluoromethylsulfonyl)methanide is used as suspension in non-coordinating solvents, preferably dichloromethane, and the reaction of alkyl ethers or alkyl acetals having a relative reactivity kᵣₑₗ, which is greater than that of benzaldehyde dimethylacetal, with nucleophiles having a nucleophilicity N ≥ 1 is catalysed.

10. Use according to Claim 1, characterized in that magnesium tris(trifluoromethylsulfonyl)methanide is used as suspension in a non-coordinating solvent, preferably in dichloromethane, and the reaction of alkyl ethers or alkyl acetals having a relative reactivity kᵣₑₗ which is greater than that of 1-tolyl-1-methoxyethane, with nucleophiles having a nucleophilicity N ≥ 1 is catalysed.

11. Use according to Claim 1, characterized in that lithium tris (trifluoromethylsulfonyl)methanide or magnesium tris (trifluoromethylsulfonyl)methanide is used as suspension in non-coordinating solvents, preferably in dichloromethane, and the reaction of aldehydes having a reactivity which is at least equal to greater than that of benzaldehyde with nucleophiles having a nucleophilicity N ≥ 5 is catalysed.

12. Use according to Claim 1, characterized in that lithium tris(trifluoromethylsulfonyl)methanide or magnesium tris (trifluoromethylsulfonyl)methanide is used as suspension in non-coordinating solvents, preferably in dichloromethane and the reaction of ketones having a reactivity which is at least equal to or greater than that of acetophenone with nucleophiles having a nucleophilicity N ≥ 9 is catalysed.

13. Use according to Claim 1, characterized in that lithium tris (trifluoromethylsulfonyl)methanide or magnesium tris(trifluoromethylsulfonyl)methanide is used and the reaction of acyl halides, in particular acyl chlorides or acyl bromides, whose reactivity is equal to or greater than that of benzoyl chloride towards nucleophiles having a nucleophilicity N ≥ 9 is catalysed.

14. Use according to Claim 1, characterized in that lithium tris (trifluoromethylsulfonyl)methanide or magnesium tris (trifluoromethylsulfonyl)methanide is used and the reaction of carboxylic anhydrides whose reactivity is equal to or greater than that of acetic anhydride towards nucleophiles having a nucleophilicity N ≥ -2 is catalysed.

15. Use according to Claim 1, characterized in that tris (trifluoromethylsulfonyl)methane is used and Friedel-Crafts acylations are catalysed.

## Revendications

1. Utilisation d'au moins un composé de formule (1) :
M^{+x}(C(SO₂CF₃)₃)ₓ (1)
où x est égal à 1 ou 2,
M représente un atome d'hydrogène ou de métal alcalin, lorsque x est égal à 1, ou un atome de métal alcalinoterreux, lorsque x est égal à 2, comme catalyseur pour des synthèses reliant C-C.

2. Utilisation selon la revendication 1 pour la réaction d'électrophiles activables par les acides de Lewis, en particulier les halogénures d'alkyles, les dialkyl-acétals, les éthers alkyliques, les N,O-acétals, les cétones, les aldéhydes, les halogénures ou anhydrides des acides carboxyliques avec des nucléophiles ou pour les cycloadditions [4+2].

3. Utilisation selon la revendication 1 ou 2, caractérisée en ce que M représente l'hydrogène, le lithium, le sodium, le potassium, le magnésium ou le calcium, en particulier l'hydrogène, le lithium ou le magnésium.

4. Utilisation selon la revendication 1 ou 2, caractérisée en ce que les composés de formule (1) sont utilisés sous forme de solution homogène dans des solvants organiques, coordinants, de préférence dans l'éther diéthylique.

5. Utilisation selon la revendication 1 ou 2, caractérisée en ce que les composés de formule (1) sont utilisés sous forme de suspension dans des solvants organiques, non coordinants, de préférence dans le dichlorométhane.

6. Utilisation selon la revendication 1, caractérisée en ce que l'on utilise le lithium tris-(trifluorométhylsulfonyl)-méthanide mis en suspension dans un solvant non coordinant, de préférence dans le dichlorométhane, comme catalyseur acide de Lewis pour la réaction des halogénures d'alkyles, de préférence les chlorures ou bromures, avec une vitesse d'éthanolyse k_{EtOH}(25°C) > 1x10⁻⁵s⁻¹ avec des nucléophiles dont la nucléophilie N est ≥ 1.

7. Utilisation selon la revendication 1, caractérisée en ce que l'on utilise le lithium tris-(trifluorométhylsulfonyl)-méthanide, dissous dans un solvant organique, coordinant, de préférence dans le diéthyléther, comme catalyseur acide de Lewis pour la réaction des halogénures d'alkyles, de préférence les chlorures ou bromures d'alkyles, avec une vitesse d'éthanolyse k_{EtOH} (25°C) > 1x10⁻³s⁻¹ avec des nucléophiles dont la nucléophilie N est ≥ 1.

8. Utilisation selon la revendication 1, caractérisée en ce que l'on utilise le magnésium tris-(trifluorométhylsulfonyl)-méthanide, mis en suspension dans un solvant non coordinant, de préférence dans le dichlorométhane, pour la réaction des halogénures d'alkyles, de préférence les chlorures ou bromures d'alkyles, avec une vitesse d'éthanolyse k_{EtOH}(25°C) > 1X10⁻⁷s⁻¹ avec des nucléophiles dont la nucléophilie N est ≥ 1.

9. Utilisation selon la revendication 1, caractérisée en ce que l'on utilise le lithium tris-(trifluorométhylsulfonyl)-méthanide sous forme de suspension dans des solvants non coordinants, de préférence le dichlorométhane, et en ce que l'on catalyse la réaction des éthers alkyliques ou des alkylacétals ayant une réactivité relative kᵣₑₗ plus grande que celle du benzaldéhyde diméthylacétal, avec des nucléophiles dont la nucléophilie N est ≥ 1.

10. Utilisation selon la revendication 1, caractérisée en ce que l'on utilise le magnésium tris-(trifluorométhylsulfonyl)-méthanide sous forme de suspension dans un solvant non coordinant, de préférence dans le dichlorométhane, et en ce que l'on catalyse la réaction des éthers alkyliques ou des alkylacétals ayant une réactivité relative kᵣₑₗ plus grande que celle du 1-tolyl-1-méthoxyéthane, avec des nucléophiles dont la nucléophilie N est ≥ 1.

11. Utilisation selon la revendication 1, caractérisée en ce que l'on utilise le lithium ou Mg tris-(trifluorométhylsulfonyl)-méthanide sous forme de suspension dans des solvants non coordinants, de préférence dans le dichlorométhane, et en ce que l'on catalyse la réaction des aldéhydes ayant une réactivité au moins égale ou plus grande que celle du benzaldéhyde, avec des nucléophiles dont la nucléophilie N est ≥ 5.

12. Utilisation selon la revendication 1, caractérisée en ce que l'on utilise le lithium ou Mg tris-(trifluorométhylsulfonyl)-méthanide sous forme de suspension dans des solvants non coordinants, de préférence dans du dichlorométhane et en ce que l'on catalyse la réaction des cétones ayant une réactivité au moins égale ou plus grande que celle de l'acétophénone, avec des nucléophiles dont la nucléophilie N est ≥ 9.

13. Utilisation selon la revendication 1, caractérisée en ce que l'on utilise le lithium ou Mg tris-(trifluorométhylsulfonyl)-méthanide et en ce que l'on catalyse la réaction des halogénures des acides carboxyliques, en particulier des chlorures ou bromures des acides carboxyliques, dont la réactivité est identique ou plus grande que celle du chlorure de benzoyle avec des nucléophiles ayant une nucléophilie N ≥ 9.

14. Utilisation selon la revendication 1, caractérisée en ce que l'on utilise le lithium ou Mg tris-(trifluorométhylsulfonyl)-méthanide et en ce que l'on catalyse la réaction des anhydrides d'acides carboxyliques dont la réactivité est identique ou plus grande que celle de l'acétanhydride, avec des nucléophiles ayant une nucléophilie N ≥ -2.

15. Utilisation selon la revendication 1, caractérisée en ce que l'on utilise le tris-(trifluorométhylsulfonyl)-méthane et en ce que l'on catalyse les acylations par Friedel-Craft.
